(19) Europäisches Patentamt
European Patent Office
Office européen des brevets

(11) EP 0 509 516 B1

(12) EUROPEAN PATENT SPECIFICATION

(45) Date of publication and mention of the grant of the patent:
26.03.1997 Bulletin 1997/13

(51) Int. Cl.$^6$: A61K 6/083

(21) Application number: 92106641.1

(22) Date of filing: 16.04.1992

(54) **Dental restoration composition**

Zahnwiederherstellungsmaterial

Composition de restauration dentaire

(84) Designated Contracting States:
DE FR GB

(30) Priority: 17.04.1991 US 686685

(43) Date of publication of application:
21.10.1992 Bulletin 1992/43

(73) Proprietor: SANKIN KOGYO KABUSHIKI KAISHA
Ohtawara-shi, Tochigi-ken (JP)

(72) Inventor: Tsunekawa, Masayoshi,
c/o Sankin Kogyo K. K.
Kawanishi-shi, Hyogo-ken (JP)

(74) Representative: VOSSIUS & PARTNER
Postfach 86 07 67
81634 München (DE)

(56) References cited:
EP-A- 0 115 410
EP-A- 0 115 948
EP-A- 0 363 903

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 509 516 B1

Printed by Rank Xerox (UK) Business Services
2.13.17/3.4

## Description

This invention relates to a light-curing fluoride releasing cement effective for filling cavities and bonding composite resin or amalgam to teeth and to achieve a strong bond and a reduced marginal microleakage.

Amalgam is an exceedingly common product used in filling tooth cavities in human beings. The main constituents of amalgam are mercury and silver. Amalgam had problems because good adhesion between the tooth and the amalgam is not obtained and also a good seal between the amalgam and the tooth is not obtained which substantially prevents ingress of mouth fluids and bacteria into the filled cavity (microleakage) and thus prevents further decay of the teeth. Thus, marginal microleakage has been an inherent problem in conjunction with amalgam restorations. Amalgam also requires cavity preparations plus usage of a retention form which can cause damage to non-carious tooth structure.

It is becoming apparent that a good number of individuals are allergic to mercury and are not able to utilize amalgam in their teeth. It has long been known that there is a need for a dental restoration composition which does not include mercury. It has been known to construct dental restoration compositions of a resin composition such as is shown and described in United States Patent No. 4,500,657 .
A similar composition is discussed within United States Patent No. 4,814,362. Improvements in dental adhesives are discussed in detail within United States Patent Numbers 4,479,782 and 4,657,941. These adhesives are commonly used as cement liners to stop microleakage under restorations. The bonding agent with the cement liner is to adhere to the dentin and reduce pulpal irritation.

Recently, four fluoride-releasing liners have been introduced in the market. These four are sold under the tradenames of TIME LINE, VITRABOND, XR IONOMER and ZIONOMER. These four liners have been developed to overcome the major disadvantage of conventional lining cements which are (1) long-setting time (three to five minutes), (2) surface dissolution upon acid etching, (3) dehydration upon drying of etched enamel and (4) low mechanical strength. These liners have achieved some success with (1), (2) and (3), but with (4) only limited success has been obtained. Actually, the mechanical strength of these liners is just not adequate for longevity of the restoration utilizing the liner.

EP-A-0 363 903 relates to a dental resin composition which comprises at least one adhesive promoting agent. Optionally the composition may include a source of elutable fluoride ions. In a preferred embodiment the composition comprises by weight about 40-60% resin monomers, 0.2-10% adhesion promoting compound, 0.05-2.0% photoinitiator, 0.2-2.0% reducing agents, 0-50% vol inorganic fillers, 1.5-2.5% sodium fluoride and 0.05-0.2% stabilizer.

EP-A-0 115 948 provides an adhesive comprising (a) 1 part by weight of a compound represented by formula (I)

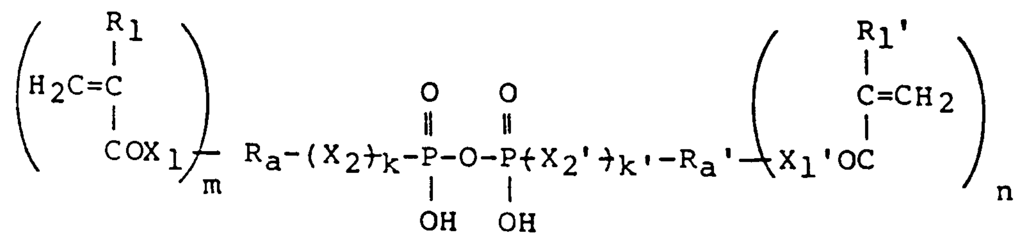

(I)

and (b) 0 to 199 parts by weight of a monomer that is copolymerizable with the compound (I).

EP-A-0 219 058 and US-P-4,872,936 relate to polymerizable cement mixtures containing

(a) polymerizable unsaturated monomers and/or oligomers and/or prepolymers containing acid groups and/or their reactive acid-derivative groups
(b) reactive fillers that can react with these acids or acid derivatives, and
(c) curing agents

that can be employed especially in dentistry as improved dental mixtures as well as in medicine.

Some lining cements require the removal of a dentin smear layer prior to usage. The smear layer is a naturally produced layer during the production of the tooth cavity and functions as a natural cavity liner. It has been thought by some that this layer must be removed for optimum bonding. The removal of this dentin smear layer generally increases hypersensitivity. The primary object of the present invention is to develop an effective formulation of a light-curing fluoride-releasing restorative material for adhesion to dentin without requiring the removal of the smear layer and to achieve substantially superior adhesion between the tooth and the filling material with the filling material being effective in substantially eliminating marginal microleakage in conjunction with the cavity within which it is applied.

One object of the present invention is to construct a dental restoration composition which can be used both as a cement liner for an amalgam or a composite restoration and as a complete material eliminating the need for the amalgam.

Another object of the present invention is to arrive at a dental filling composition that requires no enamel etching of teeth, no dentin conditioning of the teeth, no primer or bonding agent application prior to completing of the restoration.

Another object of the present invention is to produce a dental restoration composition which has significantly increased adhesive characteristics over and above prior art types of dental restoration materials.

Another object of the present invention is to construct a dental restoration composition which demonstrates significantly less microleakage at the gingival margin than prior art dental restoration materials.

The present invention provides a light-curing, fluoride-releasing, dental restoration composition according to claims 1 to 16.

The dental restoration composition of the present invention is composed of a powder and a liquid with approximately one part powder to be combined with 0.88 parts liquid by weight. A typical powder formulation would be one hundred parts by weight of strontium aluminofluorosilicate glass having an average particle size of under one micron, four parts by weight of p-toluenesulfinic acid, Na salt dihydrate and three parts by weight of 2-methacryloxyethyl p-(N,N-dimethyl)aminobenzoate. A typical formulation for the liquid would be forty parts tetramethacryloxyethyl pyrophosphate, thirty parts by weight of methacryloxyethyl phosphate, thirty parts by weight of ethoxylated bis-phenol A-dimethacrylate and 0.26 parts by weight of camphorquinone. The function of the aromatic sulfinic acid salt is that of an accelerator to function as a catalyst to cause simultaneous setting of the adhesive and the filler when the powder and the liquid are combined. The adhesive comprises a phosphate based methacrylate and a dimethacrylate. The photoinitiator comprises camphorquinone and amine. The filler comprises the glass.

The primary substance in the powder is the filler with the preferred filler material comprising strontium aluminofluorosilicate glass. It is considered to be within the scope of this invention that other fillers could be utilized such as quartz and zinc oxide or inorganic fillers or a powder of polymethacrylates and Teflon® powder or organic fillers.

The filler must be non-toxic and insoluble in saliva, and of a nature such that it imparts a workable viscosity to the adhesive composition, in other words, enabling molding and manipulation during application. Suitable fillers are typically oxidic refractory inorganic materials which are clear or white in color. Representative fillers include polymethacrylate, polyethylmethacrylate, quartz powders, silica gel, aluminosilicate, colloidalsilica, glass beads, aluminumoxide, titanium dioxide, zirconia, silicate glass and phosphate glass. The filler should contain leachable fluoride so that it releases fluoride over a prolonged period of use thereby substantially preventing decalcification of the tooth area adhered to. Particularly preferred fillers for use are the fluoride releasing silicates and the particularly preferred material is an aluminofluorosilicate glass such as strontium aluminofluorosilicate. Aluminofluorosillcate glass fillers are available commercially and can be prepared by known methods as described in United States Patent No. 4,775,592. See also, United States Patent Numbers 3,814,717, 4,360,605, and 4,376,835 for other descriptions of fluoride-containing glasses which can be used herein.

It is preferred that the filler material be in the form of relatively small particles with an average size of less than one μm. A smaller particle size has been found to give rise to better adhesion and also to a higher rate of fluoride release due to the greater surface area of the smaller particles.

Preparation of the strontium aluminofluorosilicate glass is as follows: The glass was prepared by fusing mixtures of silica, aluminophosphate, aluminum hydroxide, calcium carbonate, strontium carbonate, strontium nitrate and aluminofluoride in a platinum crucible at 1350°C, as shown in Table I. After fusion, the liquid glass was poured from the crucible and cooled rapidly. The resultant glass was dried and crushed until it passed through a 350 mesh sieve. The glass was finely ground in a ball mill to less than 1 micron using isopropyl alcohol as a medium. A silanated glass was prepared in a tumble mixer by combining 100 parts by weight of the glass and 1 part by weight of γ-methacryloxypropyltrimethoxy silane with 200 parts by weight of 0.5% acetic acid containing ethanol solution. The tumbling continued for 2 hours. When ethyl alcohol was evaporated, the glass was heated to 130°C for 3 hours. After crushing and sieving, the following glass composition (Table I) was obtained.

Table I

| The Glass Compositions | |
|---|---|
| Raw Materials | Parts by Weight |
| $SiO_2$ | 35.0 |
| $Al(PO_3)_3$ | 7.0 |
| $Al(OH)_3$ | 9.0 |
| $CaCO_3$ | 10.5 |
| $SrCO_3$ | 21.5 |
| $Sr(NO_3)_2$ | 5.0 |
| $AlF_3$ | 25.0 |

The adhesive within the composition of this invention constitutes a light-curable acrylate-based resin. Most preferably, the adhesive constitutes about forty-eight percent by weight of the overall weight of the composition. The polymerizable resin contains one or more photopolymerizable monomers with ethoxylated bis-phenol A-dimethacrylate being preferred. However, it is considered to be within the scope of this invention that there may be utilized another polymerizable resin, as a binder, selected from the group consisting of bis-phenol A-diglycidyl methacrylate, bis-phenol A-dimethacrylate, numerous other hydrophobic dimethacrylates, i.e., virtually any type of methacrylate that is usable in dental and orthodontic applications and compositions. It is to be understood that this binder is hydrophobic, meaning insoluble in water. Also, 2,2-bis[4-(3-methacryloxy-2-hydroxypropoxy) phenyl] propane could be used.

The desirable diluent is tetramethacryloxyethyl pyrophosphate. Synthesis of tetramethacryloxyethyl pyrophosphate is as follows: A mixture of 2-hydroxyethyl methacrylate (4 mols) and tri-ethylamine (4 mols) in anhydrous benzene was added dropwise to the anhydrous benzene solution of tetrachloropyrophosphate (1 mole) at -15°C with vigorous stirring. The reaction mixture was stirred at 0°C for 2 hours, and then at room temperature for 1 hour. After the reaction was complete (heat generation ceased), excessive amounts of water were added to the reaction mixture. The benzene layer was extracted, washed with 5% HCl aq, saturated $NaHCO_3$ aq and water. The benzene layer was isolated and dried with $Na_2SO_4$. Evaporation of benzene under reduced pressure gave a colorless oil of tetramethacryloxyethyl pryophosphate. A small portion of the oil was purified with alumina column chromatography using ethyl acetatehexane as solvent. The chemical structure was identified by elemental analysis, IR and NMR spectra, calculated as $C_{24}H_{36}O_{15}P_2$.

This binder is to be combined in an approximately equal amount by weight with a high viscosity (rather thick resembling conventional honey) methacryloxyethylphosphate. This monomer is dissolvable in water and therefore hydrophilic. Other hydrophilic monomers that could be utilized are methacryloxymethyl phosphate, methacryloxypropyl phosphate, methacryloxybutyl phosphate, methacryloxypentyl phosphate, methacryloxyhexyl phosphate. The general formula for this monomer (an acidic phosphor-containing methacrylate) is as follows: $CH_2=C(CH_3)-COO-R-(X)_m-P(=0)(OH)_2$ wherein R represents an alkylene group of 1-4 carbon atoms; X represents O, S or NH; and m is an integer of 0 or 1. Because of the high viscosity of this phosphate it is necessary to dilute it to make it thinner. A desirable low viscosity diluent would be tetramethacryloxyethylpyrophosphate. A typical composition of the adhesive only would be forty percent by weight of the pyrophosphate, thirty percent by weight of phosphate and thirty percent by weight of the dimethacrylate.

It has been found to be important to have a certain amount of the adhesive hydrophobic and a certain amount hydrophilic. It is to be remembered that the dental restoration composition of this invention is to be used within a tooth cavity without any prior pretreatment other than the drilling of the cavity. Inherently there will be moisture produced from the body within that cavity. If the adhesive were totally hydrophilic, the moisture would be absorbed by the adhesive but the bond strength of the adhesive would not be adequate. If the adhesive were totally hydrophobic, the moisture would act as a barrier to the adhesion and again the adhesion would not be adequate. By utilizing a proper combination of hydrophobic and hydrophilic materials, the moisture is absorbed by the hydrophilic portion of the adhesive essentially drying out the tooth cavity which causes a strong adhesive bond to occur between the dentin and enamel of the tooth and the hydrophobic components of the adhesive. As previously mentioned, the adhesive strength is approximately twice as great as the next best lining cement that is currently being sold on the market. This substantial increase in adhesive strength is a great benefit to prevent marginal microleakage fluids between the restoration and the tooth and also provide for long-term usage of the restoration composition without failure. Long-term (a plurality of years) usage is important in order to be an effective replacement for amalgam.

The composition of this invention contains an amount of a photoinitiator (an accelerator) effective to catalyze polymerization upon irradiation. Generally, the effective amount will be in the range of approximately 1.5 percent by weight relative to the total weight of the composition. The photoinitiator composition acts as a source of free radicals when the adhesive of the composition is irradiated with visible light. The photoinitiator composition of this invention contains a tertiary amine reducing agent and an α-diketone. A wide variety of tertiary amines may be used. However, preferred amines for use herein are selected from the group consisting of N,N-dimethylamino-p-toluidine, butyl diethanolamine, N,N-dimethylaminoethyl methacrylate, morpholinoethyl methacrylate, ethyl p-(N,N-dimethylamino) benzoate, 2-methacryloxyethyl p-(N,N-dimethylamino) benzoate, and dimethylamino benzoic acid or its esters. Similarly, a number of α-diketones may be used. However, preferred α-diketones for use herein are selected from the group consisting of camphorquinone, benzil, biacetyl, 9,10-phenanthrenequinone and naphthoquinone. Most preferred for incorporation into the present compositions is camphorquinone. The present photoinitiator composition contains about one and one-half percent by weight of the tertiary amine and about 0.13 percent by weight of the α-diketone.

The composition of this invention, as previously mentioned, is prepared by simply mixing homogeneously the various components with one another. The resin materials are liquid and thus provide a suitable vehicle for the powder type of filler. The powder/liquid ratio is 1.0 / 0.88 by weight. A typical time period for application of the light for curing was forty seconds per installation.

Example 1

A Typical Powder and Liquid

Formulation is as follows:

POWDER FORMULATION

| Constituent: | Parts by Weight |
|---|---|
| Strontium aluminofluorosilicate | 100 |
| p-Toluenesulfinic acid, sodium salt, dihydrate | 4 |
| Methacryloxyethyl p-(dimethylamino) benzoate | 3 |
| These were mixed in a ball mill for 3 hours. | |

LIQUID FORMULATION

| Constituent: | Parts by Weight |
|---|---|
| Tetramethacryloxyethyl pyrophosphate | 40 |
| Methacryloxyethyl phosphate | 30 |
| Ethoxylated bis-phenol A-dimethacrylate | 30 |
| Camphorquinone | 0.26 |
| These were mixed by vigorous stirring at room temperature for 24 hours. | |

SHEAR BOND STRENGTH FOR LINER

The powder and liquid were mixed in a ratio of 1/0.88 by weight. The shear bond strength to dentin was tested by the following method and compared with four known (prior art) light-curing, fluoride-releasing liners currently on the market.

TESTING METHOD

Extracted human molars stored in a 1% chloramine solution at room temperature were used as test specimens. These specimens were embedded in a fast cure acrylic resin in a cylindrical plastic mold. The crown part of each tooth was removed with a low speed saw. The exposed occlusal part of the dentin surface of the test specimens was ground wet one 800 grit silicon carbide paper on a polishing machine. The embedded teeth were washed in running fluoride-free water and dried with an oil-free air syringe. The adhesion area was defined by using an adhesive water-repellant sticker with a 3mm diameter opening in its center. A cylindrical plastic tube was placed over the 3mm opening and fixed with dental wax. The material was cured for 20 seconds with a visible light unit. A thin layer of bonding agent (Scotchbond 2 adhesive, 3M) was applied onto the liner surface and light-cured for 20 seconds. Composite resin (Silux, 3M) was then placed in the cylindrical plastic tube. The resin cylinders were light-cured for 40 seconds. The test specimens were immersed in distilled water at 37°C. After 24 hours the test specimens were clamped horizontally into the shear adhesion apparatus and loaded parallel to the vertical axis of the restorative resins. Bond strengths were measured by an Instron universal testing machine (Model 1011, Instron Corp., Canton, MA) at a cross-head speed of 1mm/min. Ten specimens were tested for each variable.

RESULTS

Shear bond strengths (MPa) of light-cured fluoride-releasing liners to human dentin after 24 hours in 37°C water:

| This Invention | Zionomer | XR Ionomer | Vitrabond | Time Line |
|---|---|---|---|---|
| 7.5 | 3.8 | 1.9 | 2.2 | 0.0 |

MICROLEAKAGE FOR LINER

The powder and liquid were mixed in ratio of 1/0.88 by weight, and test for microleakage by the following method, and again compared with the aforementioned light-curing fluoride-releasing liners in the market.

TESTING METHOD

Extracted human molars stored under the same conditions as described in the shear bond strength test were used as test specimens. All teeth were examined for areas of decalcification, caries, or restorations, and only sound teeth were selected. Eighty Class V cavities were prepared, one on the buccal and one on the lingual surface of each tooth. The preparations were approximately 1.5mm deep, 3.5mm wide, and 2.0mm high, and all were located at the cemento-enamel junction in cementum or dentin. The cavities were prepared with a No. 56 tungsten carbide fissure bur in an ultra-high-speed handpiece using a waterspray coolant. All cavosurface margins were finished to a 90° angle with hand instruments. Enamel etching agents and bonding agents were not used so it would not confound the marginal seal evaluation. The cavity preparations on the buccal surface were cement-lined with comparative commercial products in accordance with their manufacturer's instructions. After light-curing for 20 seconds, composite resin (Silux, 3M) was placed on the liner surface and then cured for 40 seconds. All the lingual surface preparations were restored with the composition of the present invention. After immersion in 37°C water for 24 hours, all restorations were finished dry with finishing disks to simulate clinical finishing.

To prevent dye penetration in areas other than the exposed margins, the teeth were sealed with nail varnish to within 2mm of the restoration margins. The tooth apices were sealed with a light-cured composite resin. The teeth were placed into separate nylon mesh bags and thermocycled together for 1000 cycles between 5°C and 55°C with a dwell time of 30 seconds at each temperature. After thermocycling was completed, the samples were placed in a 50% aqueous solution of silver nitrate for two hours in the absence of light and then thoroughly rinsed with distilled water. The test samples were placed in photodeveloper solution under a fluorescent light for 4 hours to develop the penetration pattern and then rinsed thoroughly. The teeth were sectioned longitudinally in a buccolingual direction using an Isomet saw.

The degree of dye penetration in the cavity walls was assessed separately for occlusal and gingival walls using a binocular microscope at 25X magnification. Dye penetration was scored by an examiner who had no knowledge of which material was used for the restoration. For N=O , the amount of microleakage was zero (0). For N=1, microleakage was confined to the enamel. For N= 2 microleakage was into the dentin wall. If the microleakage was well into the dentin, N=3. If the microleakage was as far as the bottom of the cavity, N=4.

RESULTS

| Microleakage (N) scores of light cured liners at gingival (dentin-cementum wall) | | | | | | |
|---|---|---|---|---|---|---|
| Light cured Ionomer | n | 0 | 1 | 2 | 3 | 4 |
| This Invention | 40 | 35 | 5 | 0 | 0 | 0 |
| Vitrabond | 16 | 10 | 6 | 0 | 0 | 0 |
| Zionomer | 8 | 0 | 1 | 3 | 2 | 2 |
| XR ionomer | 8 | 2 | 2 | 4 | 0 | 0 |
| Time Line | 8 | 0 | 0 | 0 | 4 | 4 |

| Microleakage (N) scores of light cured liners at occlusal (dentin) wall: | | | | | | |
|---|---|---|---|---|---|---|
| Light cured Ionomer | n | 0 | 1 | 2 | 3 | 4 |
| This Invention | 40 | 37 | 3 | 0 | 0 | 0 |
| Vitrabond | 16 | 10 | 6 | 0 | 0 | 0 |
| Zionomer | 8 | 8 | 0 | 0 | 0 | 0 |
| XR ionomer | 8 | 7 | 1 | 0 | 0 | 0 |
| Time Line | 8 | 6 | 0 | 0 | 1 | 1 |

Alternative Example

Using the same powder and liquid of Example 1, shear bond strength to dentin and microleakage were tested except powder/liquid ratio of 1/0.6 by weight was used.
The results were:

The shear bond strength of the bonding system to human dentin after 24 hours of immersion in 37°C water was 85.2 $Kgf/cm^2$

| Microleakage Scores | | | | | | |
|---|---|---|---|---|---|---|
| | 0 | 1 | 2 | 3 | 4 | Total Specimens |
| at gingival (dentin-cementum) wall | 15 | 3 | 2 | 0 | 0 | 20 |
| at occlusal (enamel) wall | 18 | 1 | 1 | 0 | 0 | 20 |

Example 2

POWDER FORMULATION Parts by

| Constituent: | Weight |
|---|---|
| Strontium aluminofluorosilicate | 90 |
| Calcium hydroxide | 10 |
| p-Toluenesulfinic acid, sodium salt, dihydrate | 4 |
| 2-Methacryloxyethyl p-(N,N-dimethylamino)benzoate | 3 |
| This calcium hydroxide powder was silanated with 4% $\gamma$-methacryloxy-propyl trimethoxysilane. | |

The compressive strength and pH value were measured. The powder used in these tests had the above formulation. The liquid was the same as in Example 1. Powder/liquid ratio was 1/0.88.

Compressive strength test

The specimens were prepared by filling the mixed material in the cavity, 4mm in diameter and 5mm in height, then light-curing with a light-curing unit at upper, lower and lateral surfaces each for forty seconds. Average compressive strength of six specimens was 2096.6 $Kgf/cm^2$. pH value measurement

Seven disk specimens were made using a "Teflon" ring, 15mm in diameter and 1 mm thick. These specimens were apoxymerized with a visible light-curing unit. After polymerization, a drop of water(50µl) was placed onto each disk and then the pH values were measured using pH-meter at five, ten, thirty and sixty minutes and twenty-four hours. The results were as follows:

| Time | pH |
|---|---|
| 5 min. | 4.5 |
| 10 min. | 4.9 |
| 30 min. | 5.5 |
| 60 min. | 5.9 |
| 24 hrs. | 6.9 |

Comparative Example 1

In Example 1, removal of glass filler leaving the combination of adhesive, photoinitiator and accelerator resulted in decrease of shear bond strength to 1-2 MPa.

Comparative Example 2

In Example 1, removal of sodium p-toluenesulfinate dihydrate resulted in low shear bond strength.

Comparative Example 3

In Example 1, removal of methacryloxyethyl phosphate resulted in inferior scores in the microleakage test.

Comparative Example 4

In Example 1, removal of tetramethacryloxyethyl pyrophosphate and ethoxylated bis-phenol A-dimethacrylate of the hydrophobic methacrylates resulted in the disintegration of the light-cured samples after the 24 hours of immersion in 37°C water.

Although the subject matter of this invention is discussed in conjunction with a fluoride-releasing glass particulate, it is considered to be within the scope of this invention to use a non-fluoride releasing glass particulate. The subject matter of the dental restoration composition of this invention could be utilized whether or not the glass particulate contains or does not contain fluoride.

## Claims

**1.** A light-curing, fluoride-releasing, dental restoration composition, comprising:

(a) a powder which includes a fluoride-containing filler, and
(b) a liquid including an adhesive, said adhesive being formed of a first component and a second component

said first component including a methacrylate ester of a pyrophosphate, and a hydrophilic methacrylate ester of a phosphate, and said second component including a hydrophobic methacrylate,
the combination of hydrophobic and hydrophilic compounds being such that the body moisture within the dental cavity is absorbed by the hydrophilic portion of the adhesive thereby causing an adhesive bond to occur between the dentin and enamel of the tooth and the hydrophobic compounds of the adhesive,

**2.** The dental restoration composition as defined in claim 1, wherein said fluoride-containing filler comprises particles having an average particle size of one μm or less.

**3.** The composition as defined in claim 1 or 2, wherein the fluoride-containing filler is a glass filler.

**4.** The dental restoration composition as defined in claim 3, wherein said glass filler comprises strontium aluminofluoro silicate glass.

**5.** The composition as defined in any one of claims 1 to 4, wherein the hydrophilic methacrylate ester of a phosphate is a compound of the general formula:

$$CH_2=C(CH_3)\text{-}COO\text{-}R\text{-}(X)_m\text{-}P(=O)(OH)_2$$

wherein:

R represents an alkylene group of 1 to 4 carbon atoms; X represents O, S or NH; m is an integer of 0 or 1.

**6.** The dental restoration composition as defined in claim 5, wherein the hydrophilic methacrylate ester of a phosphate within said first component of the adhesive is selected from the group consisting of methacryloxymethylphosphate, methacryloxyethylphosphate, methacryloxypropylphosphate, and methacryloxybutylphosphate.

**7.** The dental restoration composition as defined in any one of claims 1 to 6, wherein said second component of the adhesive comprises a dimethacrylate.

**8.** The dental restoration composition as defined in claim 7, wherein said dimethacrylate is selected from the group consisting of ethoxylated bis-phenol A-dimethacrylate, bis-phenol A-diglycidylmethacrylate and bis-phenol A-dimethacrylate.

**9.** The dental restoration composition as defined in any one of claims 1 to 8, wherein the composition comprises a polymerization accelerator and a photoinitiator.

**10.** The composition as defined in claim 9, wherein said photoinitiator is camphorquinone and an amine.

**11.** The composition as defined in claim 10, wherein the amine is a tertiary amine.

**12.** The dental restoration composition as defined in claim 11, wherein said tertiary amine is selected from the group

consisting of N,N-dimethylamino-p-toluidine, ethyl p-(N,N-dimethylamino)benzoate and 2-methacryloxyethyl p-(N,N-dimethylamino)benzoate.

**13.** The dental restoration composition as defined in claim 9, wherein said polymerization accelerator is an aromatic sulfinic acid or its salt.

**14.** The dental restoration composition as defined in any one of claims 1 to 13, wherein said adhesive comprising 30 to 70% by weight of said first component and 30 to 70% by weight of said second component, based on the total amount by weight of said adhesive.

**15.** The dental restoration composition as defined in claim 14, wherein there is approximately two parts by weight of said first component to one part by weight of said second component contained within said adhesive.

**16.** The dental restoration composition as defined in claim 1, wherein said powder comprising strontium aluminofluoro silicate glass and p-toluene-sulfinic acid, Na salt, dihydrate and methacryloxyethyl p-(N,N-dimethylamino)benzoate; and said liquid comprising tetramethacryloxyethylpyrophosphate, methacryloxyethylphosphate, ethoxylated bis-phenol A-dimethacrylate and camphorquinone.

**Patentansprüche**

**1.** Durch Licht härtbares, Fluorid freisetzendes Zahnwiederherstellungsmaterial, umfassend

(a) ein Pulver, das einen Fluorid enthaltenden Füllstoff beinhaltet, und
(b) eine Flüssigkeit, die ein Klebemittel beinhaltet, das aus einem ersten Bestandteil und einem Zweiten Bestandteil gebildet wird,

wobei der erste Bestandteil einen Methacrylatester eines Pyrophosphats und einen hydrophilen Methacrylatester eines Phosphats und der zweite Bestandteil ein hydrophobes Methacrylat umfaßt, wobei die Kombination aus hydrophoben und hydrophilen Verbindungen dergestalt ist, daß die Körperfeuchtigkeit in der Dentalkavität durch den hydrophilen Anteil des Klebemittels absorbiert wird, wodurch eine Klebebindung zwischen dem Dentin und dem Zahnschmelz des Zahnes und den hydrophoben Verbindungen des Klebemittels ausgebildet wird.

**2.** Zahnwiederherstellungsmaterial nach Anspruch 1, in dem der Fluorid enthaltende Füllstoff Teilchen mit einer mittleren Teilchengröße von 1 µm oder weniger umfaßt.

**3.** Material nach Anspruch 1 oder 2, in dem der Fluorid enthaltende Füllstoff ein Glasfüllstoff ist.

**4.** Zahnwiederherstellungsmaterial nach Anspruch 3, in dem der Glasfüllstoff Strontium-Aluminium-Fluorsilikatglas umfaßt.

**5.** Material nach einem der Ansprüche 1 bis 4, in dem der hydrophile Methacrylatester eines Phosphats eine Verbindung der allgemeinen Formel

$$CH_2=C(CH_3)\text{-}COO\text{-}R\text{-}(X)_m\text{-}P(=O)(OH)_2,$$

bedeutet, in der R einen Alkylenrest mit 1 bis 4 Kohlenstoffatomen bedeutet, X ein Sauerstoffatom, ein Schwefelatom oder eine NH-Gruppe bedeutet und m 0 oder die ganze Zahl 1 ist.

**6.** Zahnwiederherstellungsmaterial nach Anspruch 5, in dem der hydrophile Methacrylatester eines Phosphats im ersten Bestandteil des Klebemittels aus Methacryloxymethylphosphat, Methacryloxyethylphosphat, Methacryloxypropylphosphat und Methacryloxybutylphosphat ausgewählt wird.

**7.** Zahnwiederherstellungsmaterial nach einem der Ansprüche 1 bis 6, in dem der zweite Bestandteil des Klebemittels ein Dimethacrylat umfaßt.

**8.** Zahnwiederherstellungsmaterial nach Anspruch 7, in dem das Dimethacrylat aus ethoxyliertem Bisphenol-A-Dimethacrylat, Bisphenol-A-Diglycidylmethacrylat und Bisphenol-A-Dimethacrylat ausgewählt wird.

**9.** Zahnwiederherstellungsmaterial nach einem der Ansprüche 1 bis 8, in dem das Material einen Polymerisationsbeschleuniger und einen Photoinitiator umfaßt.

**10.** Material nach Anspruch 9, in dem der Photoinitiator Kampferchinon und ein Amin ist.

**11.** Material nach Anspruch 10, in dem das Amin ein tertiäres Amin ist.

**12.** Zahnwiederherstellungsmaterial nach Anspruch 11, in dem das tertiäre Amin aus N,N-Dimethylamino-p-toluidin, Ethyl-p-(N,N-Dimethylamino)-benzoat und 2-Methacryloxyethyl-p-(N,N-dimethylamino)-benzoat ausgewählt wird.

**13.** Zahnwiederherstellungsmaterial nach Anspruch 9, in dem der Polymerisationsbeschleuniger eine aromatische Sulfinsäure oder deren Salz ist.

**14.** Zahnwiederherstellungsmaterial nach einem der Ansprüche 1 bis 13, in dem das Klebemittel 30 bis 70 Gew.-% des ersten Bestandteils und 30 bis 70 Gew.-% des zweiten Bestandteils, bezogen auf das Gesamtgewicht des Klebemittels, umfaßt.

**15.** Zahnwiederherstellungsmaterial nach Anspruch 14, in dem das Klebemittel den ersten Bestandteil und den zweiten Bestandteil im Verhältnis von etwa 2 Gew.-Teilen zu 1 Gew.-Teil enthält.

**16.** Zahnwiederherstellungsmaterial nach Anspruch 1, in dem das Pulver Strontium-Aluminium-Fluorsilikatglas, p-Toluolsulfinsäure, Natriumsalz, Dihydrat, und Methacryloxyethyl-p-(N,N-dimethylamino)-benzoat umfaßt und
die Flüssigkeit Tetramethacryloxyethylpyrophosphat, Methacryloxyethylphosphat, ethoxyliertes Bisphenol-A-dimethacrylat und Kampferchinon umfaßt.

## Revendications

**1.** Composition de réparation dentaire libérant un fluorure et durcissant à la lumière, comprenant :

(a) une poudre comprenant une charge contenant un fluorure, et
(b) un liquide comprenant un adhésif, ledit adhésif étant formé d'un premier composant et d'un second composant,

ledit premier composant comprenant un ester méthacrylate d'un pyrophosphate et un ester méthacrylate hydrophile d'un phosphate, et ledit second composant comprenant un méthacrylate hydrophobe,
la combinaison des composés hydrophobes et des composés hydrophiles étant telle que l'humidité du corps à l'intérieur de la cavité dentaire est absorbée par la partie hydrophile de l'adhésif, provoquant ainsi la formation d'une liaison adhésive entre la dentine et l'émail de la dent et les composés hydrophobes de l'adhésif.

**2.** Composition de réparation dentaire selon la revendication 1, dans laquelle ladite charge contenant un fluorure comprend des particules ayant une dimension moyenne d'au plus 1 $\mu$m.

**3.** Composition selon la revendication 1 ou 2, dans laquelle la charge contenant un fluorure est une charge en verre.

**4.** Composition de réparation dentaire selon la revendication 3, dans laquelle ladite charge en verre comprend un verre d'aluminofluorosilicate de strontium.

**5.** Composition selon l'une quelconque des revendications 1 à 4, dans laquelle l'ester méthacrylate hydrophile d'un phosphate est un composé répondant à la formule générale

$$CH_2=C(CH_3)\text{-}COO\text{-}R\text{-}(X)_m\text{-}P(=O)(OH)_2$$

où :

R représente un groupe alkylène ayant de 1 à 4 atomes de carbone ; X représente O, S ou NH ; et m est un nombre entier de 0 ou 1.

**6.** Composition de réparation dentaire selon la revendication 5, dans laquelle l'ester méthacrylate hydrophile d'un phosphate dudit premier composant de l'adhésif est choisi dans l'ensemble constitué par le phosphate de méthacryloxyméthyle, le phosphate de méthacryloxyéthyle, le phosphate de méthacryloxypropyle et le phosphate de

méthacryloxybutyle.

7. Composition de réparation dentaire selon l'une quelconque des revendications 1 à 6, dans laquelle ledit second composant de l'adhésif comprend un diméthacrylate.

8. Composition de réparation dentaire selon la revendication 7, dans laquelle ledit diméthacrylate est choisi dans l'ensemble constitué par le diméthacrylate de bisphénol A éthoxylé, le diglycidylméthacrylate de bisphénol A et le diméthacrylate de bisphénol A.

9. Composition de réparation dentaire selon l'une quelconque des revendications 1 à 8, comprenant un accélérateur de polymérisation et un photo-initiateur.

10. Composition selon la revendication 9, dans laquelle ledit photo-initiateur est la camphoquinone et une amine.

11. Composition selon la revendication 10, dans laquelle l'amine est une amine tertiaire.

12. Composition de réparation dentaire selon la revendication 11, dans laquelle ladite amine tertiaire est choisie dans l'ensemble constitué par la N,N-diméthylamino-p-toluidine, le p-(N,N-diméthylamino)benzoate d'éthyle et le p-(N,N-diméthylamino)benzoate de 2-méthacryloxyéthyle.

13. Composition de réparation dentaire selon la revendication 9, dans laquelle ledit accélérateur de polymérisation est un acide sulfinique aromatique ou son sel.

14. Composition de réparation dentaire selon l'une quelconque des revendications 1 à 13, dans laquelle ledit adhésif comprend de 30 à 70 % en poids dudit premier composant et de 30 à 70 % en poids dudit second composant, par rapport à la quantité totale en poids dudit adhésif.

15. Composition de réparation dentaire selon la revendication 14, dans laquelle ledit adhésif contient environ deux parties en poids dudit premier composant pour une partie en poids dudit second composant.

16. Composition de réparation dentaire selon la revendication 1, dans laquelle ladite poudre comprend un verre d'aluminofluorosilicate de strontium et de l'acide p-toluènesulfinique, un sel de sodium, un dihydrate et du p-(N,N-diméthylamino)benzoate de méthacryloxyéthyle ; et ledit liquide comprend du pyrophosphate de tétraméthacryloxyéthyle, du phosphate de méthacryloxyéthyle, du diméthacrylate de bisphénol A éthoxylé et de la camphoquinone.